# EUROPÄISCHE PATENTSCHRIFT

(11) **EP 1 488 209 B1**
(45) Veröffentlichungstag und Bekanntmachung des Hinweises auf die Patenterteilung: **07.12.2005**
(21) Anmeldenummer: 04700013.8
(22) Anmeldetag: 29.01.2004
(51) Int. Cl.: G01N 1/00, G01N 33/74, G01N 33/68

(54) **BESTIMMUNG EINES MIDREGIONALEN PROADRENOMEDULLIN-TEILPEPTIDS IN EINER BIOLOGISCHEN FLÜSSIGKEIT ZU DIAGNOSTISCHEN ZWECKEN SOWIE IMMUNOASSAYS FÜR DIE DURCHFÜHRUNG EINER SOLCHEN BESTIMMUNG**
IDENTIFYING A MIDREGIONAL PROADRENOMEDULLIN PARTIAL PEPTIDE IN BIOLOGICAL LIQUIDS FOR DIAGNOSTIC PURPOSES, AND IMMUNOASSAYS FOR CONDUCTING AN IDENTIFICATION OF THIS TYPE
MISE EN EVIDENCE D'UN PEPTIDE PARTIEL PROADRENOMEDULLINE A REGION MOYENNE DANS DES LIQUIDES BIOLOGIQUES, A DES FINS DIAGNOSTIQUES ET DOSAGES IMMUNOLOGIQUES UTILISES POUR EFFECTUER UNE MISE EN EVIDENCE DE CE TYPE

(30) Priorität: 10.04.2003 DE 10316583
(43) Veröffentlichungstag der Anmeldung: 22.12.2004
(73) Patentinhaber: B.R.A.H.M.S Aktiengesellschaft, 16761 Hennigsdorf (DE)
(72) Erfinder: BERGMANN, Andreas, 12351 Berlin (DE); STRUCK, Joachim, 12161 Berlin (DE)
(74) Vertreter: Andrae, Steffen
(86) Internationale Anmeldenummer: PCT/EP2004/000806
(87) Internationale Veröffentlichungsnummer: WO 2004/090546

(56) Entgegenhaltungen:
- WO-A-00/22439
- WO-A-00/69900

## Beschreibung

Die Erfindung betrifft Verfahren zur Bestimmung eines midregionalen Teilpeptids des Proadrenomedullin (mid-proAM), insbesondere die Bestimmung des proAM (45-92) Teilpeptids in biologischen Flüssigkeiten für Zwecke der medizinischen Diagnostik, und zwar insbesondere in der Sepsisdiagnostik, aber auch z.B. in der Krebsdiagnostik und Kardialdiagnostik bzw. generell im Rahmen der Diagnostik solcher Krankheitszustände, bei denen eine Bestimmung des Peptids Adrenomedullin (AM) diagnostisch relevante Ergebnisse liefert. Die erfindungsgemäßen Bestimmungen erfolgen insbesondere mittels Immunoassays eines Typs, bei dem mit einem markierten Antikörper gearbeitet wird (Sandwichassay; kompetitiver Assay nach dem SPALT oder SPART-Prinzip).

In dieser Beschreibung wird dabei der Begriff "Diagnostik" grundsätzlich als vereinfachender Oberbegriff verwendet, der auch Prognostik/Frühprognostik und therapiebegleitende Verlaufskontrolle einschließen soll.

Das Peptid Adrenomedullin (AM) wurde erstmals 1993 als neues hypotensives Peptid aus 52 Aminosäuren, das aus einem menschlichen Phänochromozytom isoliert worden war, von Kitamura et al. (vgl. 18; Zahlenangaben beziehen sich auf die beigefügte Literaturliste) beschrieben. Im gleichen Jahr wurde auch die für ein Vorläuferpeptid aus 185 Aminosäuren kodierende cDNA und die vollständige Aminosäuresequenz dieses Vorläuferpeptids beschrieben (19; SEQ ID NO:1). Das Vorläuferpeptid, das u.a. eine Signalsequenz von 21 Aminosäuren am N-Terminus umfaßt, wird als "Präproadrenomedullin" (pre-proAM) bezeichnet. In der vorliegenden Beschreibung beziehen sich alle angegebenen Aminosäuren-Positionen normalerweise auf das 185 Aminosäuren umfassende pre-proAM, das die Sequenz gemäß SEQ ID NO:1 aufweist, es sei denn, es ergibt sich aus dem konkreten Textzusammenhang etwas anderes.

Das Peptid Adrenomedullin (AM) ist ein 52 Aminosäuren umfassendes Peptid (SEQ ID NO:2), das die Aminosäuren 95 bis 146 des pre-proAM umfaßt, aus dem es durch proteolytische Spaltung gebildet wird. Von den bei der Spaltung des pre-proAM gebildeten Peptid-Fragmenten wurden bisher im wesentlichen nur wenige Fragmente genauer charakterisiert, und zwar die physiologisch wirksamen Peptide Adrenomedullin (AM) und "PAMP", ein Peptid aus den 20 Aminosäuren (22-41), die im pre-proAM auf die 21 Aminosäuren des Signalpeptids folgen. Sowohl von AM als PAMP wurden außerdem auch physiologisch aktive Unterfragmente entdeckt und näher untersucht.

Die Entdeckung und Charakterisierung des AM im Jahre 1993 löste eine intensive Forschungstätigkeit und eine Flut von Publikationen aus, deren Ergebnisse in jüngerer Zeit in verschiedenen Review-Artikeln zusammengefaßt wurden, wobei im Rahmen der vorliegenden Beschreibung insbesondere hingewiesen wird auf die in einer AM gewidmeten Nummer von "Peptides" (Peptides 22 (2001)) zu findenden Artikel, insbesondere (12) und (2). Ein weiterer Review ist (3). Bei den bisherigen wissenschaftlichen Untersuchungen wurde u.a. gefunden, dass AM als multifunktionelles regulatorisches Peptid angesehen werden kann. Es wird in einer durch Glycin verlängerten inaktiven Form in die Zirkulation freigesetzt (5). Ferner existiert ein für AM spezifisches Bindeprotein (11), das wahrscheinlich ebenfalls die Wirkung des AM moduliert.

Diejenigen physiologischen Wirkungen des AM, wie auch des PAMP, die bei den bisherigen Untersuchungen im Vordergrund standen, waren den Blutdruck beeinflussende Wirkungen. So ist AM ein wirksamer Vasodilatator, wobei die hypotensive Wirkung insbesondere Peptidabschnitten im C-terminalen Teil des AM zugeordnet werden kann. Peptidsequenzen vom N-Terminus des AM zeigen dagegen hypertensive Wirkungen (vgl. z.B. (6)).

Es wurde ferner gefunden, dass das aus dem pre-proAM gebildete o.g. weitere physiologisch aktive Peptid PAMP ebenfalls eine hypotensive Wirkung zeigt, auch wenn es einen anderen Wirkmechanismus als AM aufzuweisen scheint (vgl. neben den o.g. Review-Artikeln (2) und (3) auch (8), (9) oder (14) sowie EP 0 622 458 A2).

Es wurde ferner festgestellt, dass die Konzentrationen des AM, die man in der Zirkulation und anderen biologischen Flüssigkeiten messen kann, bei einer Reihe von Krankheitszuständen signifikant über den bei gesunden Kontrollpersonen zu findenden Konzentrationen liegen. So sind die AM-Spiegel bei Patienten mit Stauungsinsuffizienz (congestive heart failure), Myokardinfarkt, Nierenerkrankungen, Bluthochdruckerkrankungen, Diabetes mellitus, in der akuten Phase eines Schocks und bei Sepsis und septischem Schock signifikant, wenn auch in unterschiedlichem Maße erhöht (vgl. z.B. (2), Kapitel 7., und die dazu zitierte Literatur). Auch die PAMP-Konzentrationen sind bei einigen der genannten Krankheitszustände erhöht, wobei allerdings die Plasmaspiegel gegenüber AM relativ erniedrigt sind ((2); S.1702).

Es ist ferner bekannt, dass bei Sepsis bzw. beim septischen Schock ungewöhnlich hohe Konzentrationen an AM zu beobachten sind (vgl. (2) sowie (4), (1), (13), (15) und (16)). Die Befunde werden zu den typischen hämodynamischen Veränderungen in Beziehung gesetzt, die als typische Erscheinungen des Krankheitsverlaufs bei Patienten mit Sepsis und anderen schweren Krankheitsbildern wie z.B. SIRS bekannt sind.

Obwohl davon ausgegangen wird, dass AM und PAMP aus dem gleichen Vorläuferpeptid, dem pre-proAM (SEQ ID NO:1) gebildet werden, in dem die diesen Peptiden entsprechenden Aminosäuresequenzen als Teilpeptide in äquimolaren Mengen vorhanden sind, sind die in biologischen Flüssigkeiten messbaren Konzentrationen von AM bzw. PAMP anscheinend verschieden. Das ist nichts Ungewöhnliches. So können die messbaren Konzentrationen verschiedener Abbauprodukte ein- und desselben Vorläuferpeptids z.B. deshalb verschieden sein, weil sie das Ergebnis verschiedener konkurrierender Abbauwege sind, die z.B. bei unterschiedlichen Krankheitszuständen zu einer unterschiedlichen Fragmentierung eines Vorläuferpeptides und damit zu verschiedenen Abbauprodukten führen. Bestimmte im Vorläuferpeptid enthaltene Teilpeptide können dabei als freie Peptide gebildet oder auch nicht gebildet werden, und/oder verschiedene Peptide werden auf verschiedene Weise und in verschiedenen Mengen gebildet. Auch wenn zur Prozessierung eines Vorläuferpeptids nur ein einziger Abbauweg beschritten wird, und somit alle Abbauprodukte aus ein und demselben Vorläuferpeptid stammen und an sich primär in äquimolaren Mengen entstanden sein müssen, können die in biologischen Flüssigkeiten messbaren Stationärkonzentrationen verschiedener Teilpeptide und Fragmente sehr verschieden sein, nämlich z.B. dann, wenn einzelne von ihnen mit unterschiedlicher Geschwindigkeit gebildet werden und/oder in der jeweiligen biologischen Flüssigkeit unterschiedliche individuelle Stabilitäten (Lebensdauern) aufweisen, oder wenn sie aufgrund unterschiedlicher Clearance-Mechanismen und/oder mit unterschiedlichen Clearance-Geschwindigkeiten aus der Zirkulation entfernt werden.

So kann man im Zusammenhang mit der Bildung von AM zwar davon ausgehen, dass bei der proteolytischen Prozessierung von pre-proAM neben AM und PAMP auch noch andere Peptid-fragmente gebildet werden müssen. Es sind jedoch in der wissenschaftichen Literatur keinerlei Daten zum Auftreten und zur Stabilität derartiger möglicher weiterer Fragmente zu finden, und das, obwohl zu Forschungszwecken z.B. von der Fa. Phoenix Pharmaceuticals, Inc., derartigen pre-proAM Peptidfragmenten entsprechende Peptide und auch Radioimmunoassays (RIA) zu deren Bestimmung kommerziell angeboten werden.

Aus WO00/69900 ist ein Peptid bekannt (SEQ ID NO:939), das dem der SEQ ID NO:3 der vorliegenden Anmeldung entspricht. Allerdings enthüllt WO00/69900 keine diagnostische Anwendung dieses Peptids.

Aufbauend auf Erkenntnissen, die mit dem Auftreten des Prohormons Procalcitonin bei Sepsis gewonnen worden waren (vgl. z.B. EP 0 656 121 B1), und ausgehend von der Hypothese, dass bei Sepsis möglicherweise auch andere normalerweise nicht zu beobachtende Prohormone in der Zirkulation von Sepsispatienten nachzuweisen sein könnten, wurde von der Anmelderin unter Verwendung eines kommerziell erhältlichen RIA mit einem Antikörper, der an die Aminosäuren 45-92 des pre-proAM bindet, jedoch nicht an Sequenzen des reifen AM, ein orientierender Versuch zum Nachweis von Proadrenomedullin in Seren von Sepsispatienten durchgeführt. Die Ergebnisse, die in der Veröffentlichung WO 00/22439 beschrieben werden, belegen eine gegenüber gesunden Kontrollpersonen erhöhte Konzentration eines vorläufig als Proadrenomedullin bezeichneten Analyten. Die gemessene Erhöhung lag jedoch nur in der Größenordnung von etwa dem Doppelten des Normalwerts; war also relativ geringfügig. Angesichts von Literaturdaten, die bei Sepsis von erhöhten AM-Werten in der Größenordnung des 12fachen Normalwerts berichten, erschien die beobachtete Erhöhung auf etwa das Doppelte des Normalwerts für die mit dem verwendeten Assay gemessene proAM-Immunreaktivität wenig attraktiv, im Rahmen der Sepsisdiagnostik anstelle von AM diese "proAM-Immunreaktivität" zu bestimmen. Ob bei dem beschriebenen Versuch tatsächlich Proadrenomedullin (22-185 bzw. 22-146) gemessen wurde, oder ob die auf die beschriebene Weise gemessene Proadrenomedullin-Immunreaktivität auf eine oder mehrere andere in den Patientenproben auftretende Spezies zurückzuführen war, war auf der Basis der gemessenen Befunde nicht entscheidbar.

Im Rahmen ihrer umfassenden Forschungs- und Entwicklungsarbeiten zu Biomarkern, die für die Sepsisdiagnostik von klinischem Nutzen sein können, und insbesondere auch im Hinblick auf das Ziel, auf dem Wege einer Multiparameter-Bestimmung (gleichzeitige Bestimmung mehrerer Biomarker) die Sepsis-Feindiagnostik verbessern zu können, zog die Anmelderin auch die Bestimmung bzw. zusätzliche Bestimmung des bei Sepsis erhöhten AM in Betracht. Wie sich jedoch zeigte, war eine zuverlässige Bestimmung von AM unter Gewinnung von Resultaten, die einen einfachen Vergleich von Messergebnissen über die Grenzen der jeweiligen einzelnen Forschungsarbeit hinaus ermöglichen würden, nicht ohne weiteres möglich. Die Daten der meisten Forschungsarbeiten wurden mit RIAs gewonnen, die auf einer Konkurrenz von AM mit einem markierten Markerpeptid um eine gemeinsame AM-Bindungsstelle eines Antikörpers beruhten. Die jeweiligen RIAs waren oft individuelle Entwicklungen, und es wurde mit verschiedenen Antikörpern und Peptiden gearbeitet, was einen quantitativen Interassay-Vergleich der erhaltenen Messergebnisse erschwerte (vgl. z.B. 10). Außerdem hatten jüngere Forschungsergebnisse gezeigt, dass verschiedene Formen von AM (mit oder ohne C-terminalem Glycinrest) existierten, denen unterschiedliche Aktivitäten zugeordnet werden konnten (vgl. (2) sowie z.B. (5)). Die Entdeckung eines Bindeproteins (vgl. 11) für AM führte zu einer weiteren Verkomplizierung der Situation - sowohl der vorhandene oder fehlende Glycinrest, als auch die fehlende oder vorhandene Komplexierung von AM durch sein Bindeprotein können die Bestimmung von AM in Abhängigkeit vom jeweiligen Assay unvorhersehbar beeinflussen. Diese Umstände stellen hohe Anforderungen an einen validen Immunoassay für AM, der für Routineuntersuchungen geeignet ist: Für einen solchen Assay müssen geeignete Antikörper gefunden werden, die an solche AM-Bereiche binden, die nicht durch das Bindeprotein belegt sind - wenn es solche Bereiche überhaupt gibt. Oder es muß ein vorausgehender Schritt der Freisetzung und Abtrennung des Bindeproteins vom AM durchgeführt werden, wobei der Einfluß eines solchen Schritts auf die Stabilität des AM und/oder die erhältlichen Messwerte nur schwer abschätzbar ist. Dass neben dem kompletten AM physiologisch auch verschiedene AM-Teilpeptide gefunden werden und im physiologischen Gesamtgeschenen eine Rolle zu spielen scheinen, erschwert die Schaffung eines validen Immunoassays und die Vergleichbarkeit von der Literatur entnehmbaren Meßwerten weiter.

Die Anmelderin hat sich daher die Aufgabe gestellt, ein valides, routinetaugliches Meßverfahren zu schaffen, das gegenüber den o.g. Störeinflüssen einer direkten Messung von AM weitgehend unempfindlich ist und das zuverlässige Werte für die physiologische Produktion von AM und/oder seiner Vorläufer bei verschiedenen Krankheitszuständen, insbesondere Sepsis oder anderen Krankheitszuständen, bei denen erhöhte Werte für AM gefunden werden, liefern kann.

Diese Aufgabe wird erfindungsgemäß dadurch gelöst, dass zu Diagnosezwecken nicht AM oder ein anderes der bisher untersuchten pre-proAM Teilpeptide bestimmt wird, sondern ein midregionales Teilpeptid, das die Aminosäuren 42-95 des'pre-proAM enthält (SEQ ID NO:3), wobei die Bestimmung besonders bevorzugt mit einem Immunoassay erfolgt, bei dem mit einem markierten Antikörper gearbeitet wird.

Anspruch 1 gibt den Kern der vorliegenden Erfindung wieder.

Vorteilhafte und derzeit bevorzugte Ausgestaltungen der Erfindung sind den Unteransprüchen zu entnehmen.

Zur Lösung der Aufgabe, ein Bestimmungsverfahren zu schaffen, das die Bildung von AM bzw. seiner Vorläufer- oder Nebenprodukte bei verschiedenen Krankheitszuständen, insbesondere Sepsis, aber auch z.B. Kardialerkrankungen, Bluthochdruckerkrankungen oder Krebserkrankungen oder anderen Erkrankungen, bei denen erhöhte AM-Spiegel beobachtet werden können, zuverlässig erfaßt, wurde, trotz der eher wenig vielversprechenden Ergebnisse, einerseits an das in der WO 00/22439 beschriebene Ergebnis einer bei Sepsis erhöhten "Proadrenomedullin-Immunreaktivität" angeknüpft. Andererseits wurden ergänzende umfangreiche klinische Studien unter Vermessung von Seren von Sepsis-, Krebs- und Kardialpatienten mit verschiedenen neuen Assays durchgeführt, die überraschender Weise Messwerte mit deutlich verbesserter Validität lieferten.

Die durchgeführten Untersuchungen und die signifikantesten Ergebnisse dieser Untersuchungen werden nachfolgend genauer erläutert, wobei auf Figuren Bezug genommen wird.

Dabei zeigen:

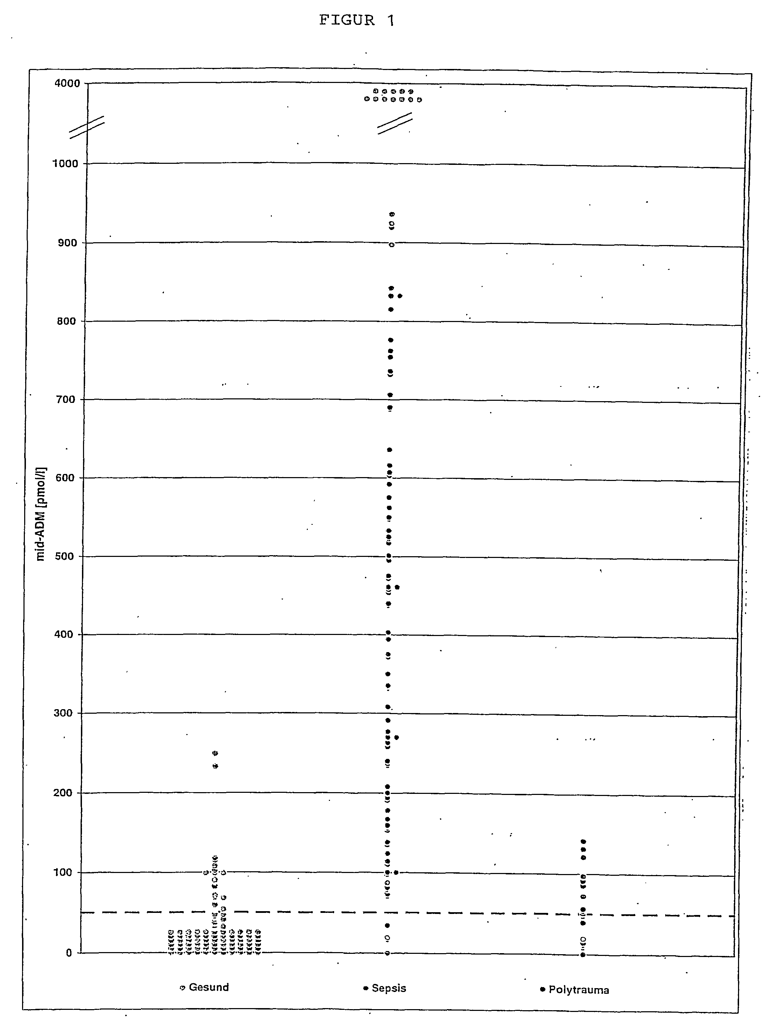

- Figur 1: die Ergebnisse der Messung von mid-proAM in Seren von 109 gesunden Normalpersonen, gegenübergestellt den Ergebnissen der Messung von 110 Seren von Septikern sowie von 20 Seren von Patienten mit Polytrauma. Alle Messungen wurden mit einem SPALT-Assay vorgenommen, wie er im experimentellen Teil genauer beschrieben wird. Bemerkenswert ist, dass - anders als im Falle von Procalcitonin und anderen Entzündungsmarkern - nur die Werte für die Sepsispatienten (hohe Konzentrationen von ca. 550 pmol/l bzw. 550 fmol/ml gegenüber Werten von 33 pmol/l für Gesunde) erhöht waren, nicht jedoch die Werte für Polytrauma-Patienten.

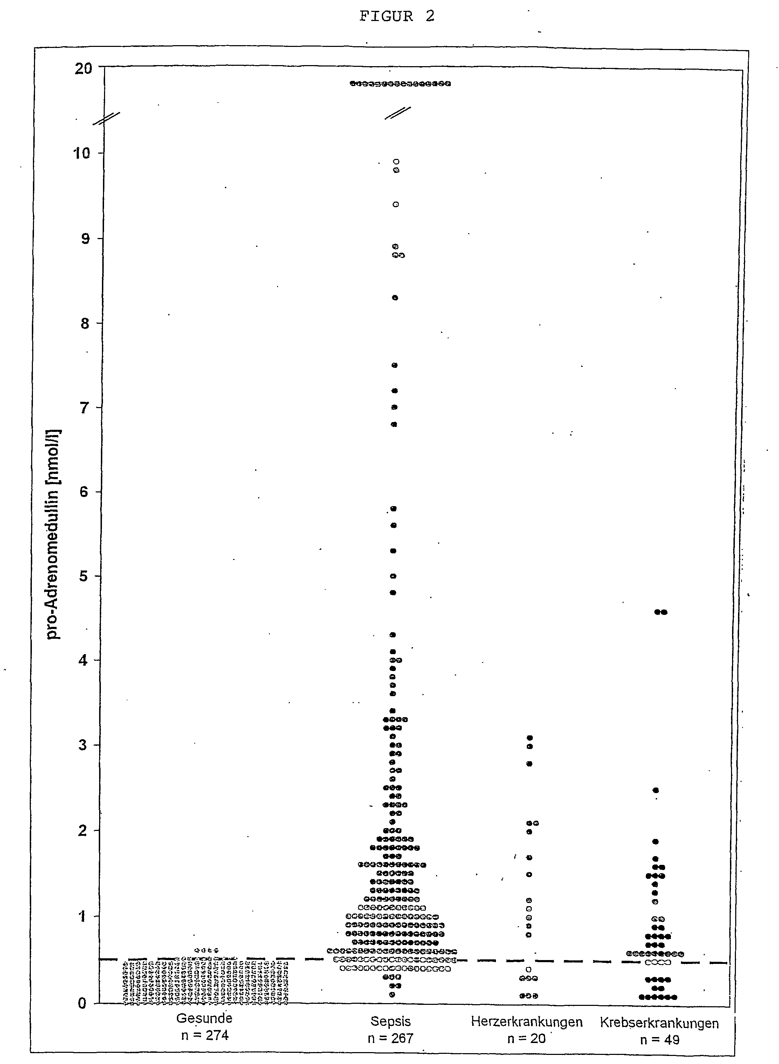

- Figur 2: die Ergebnisse der Messung von mid-proAM in Seren von 274 gesunden Normalpersonen, von 267 Septikern, von 20 Patienten mit Herzerkrankungen und 49 Krebspatienten mit einem Sandwichassay, wie er im experimentellen Teil näher beschrieben ist.

Im Zusammenhang mit den Untersuchungen, die zu der vorliegenden Erfindung führten, rückte die Frage nach der Natur der bei den verschiedenen Erkrankungen gemessenen Spezies, bzw. nach der Wahl einer besonders geeigneten Spezies für AM/proAM- Messungen, in den Vordergrund des Interesses. Da RIAs prinzipiell wenig geeignet sind, wertvolle Erkenntnisse zu dieser Frage zu liefern, und außerdem RIAs aus verschiedenen Gründen auch für das angestrebte Entwicklungsziel der Schaffung eines validen Assays für Routinebestimmungen wenig aussichtsreich erschienen, mußten zuerst neue Assays entwickelt werden, wobei Immunoassays eines Typs gewählt wurden, bei denen mit markierten Antikörpern gearbeitet werden konnte.

Im Rahmen der Untersuchung der Frage, ob die gemäß der WO 00/22439 gemessenen erhöhten Werte für eine Proadrenomedullin-Immunreaktivität bei Sepsis tatsächlich erhöhte Proadrenomedullin-Konzentrationen in den untersuchten Proben widerspiegeln, wurde zuerst ein Sandwichassay entwickelt, der aufgrund des Assaydesigns weitgehend spezifisch für Proadrenomedullin (22-146 bzw. 22-185) war, indem er weder AM noch pre-proAM-Teilpeptide, die kein AM enthielten, erkennen konnte.

Bei diesem Sandwichassay wurde mit zwei unterschiedlichen Antikörpern gearbeitet, die spezifisch die Aminosäuresequenz des Peptids (69-86: Peptidbereich SPCD19; SEQ ID NO:4) bzw. eines Peptids (129-147; C-terminales AM-Peptid) erkannten. Als standarmaterial diente das rekombinante vollständige Proadrenomedullin (22-185), das in einem kommerziellen kompetitiven AM-Assay kalibriert worden war.

Bei der Vermessung von Seren von gesunden Normalpersonen und von Sepsispatienten wurden mit diesem Sandwichassay keine erhöhten Messwerte über dem Detektionslimit von ca. 40 pg/ml erhalten (Ergebnisse nicht gezeigt). Aus diesen Befunden mußte der Schluß gezogen werden, dass die bei Sepsis gefundene erhöhte proAM-Immunreaktivität nicht auf die Anwesenheit des Proadrenomedullin-Peptids in den Proben zurück zu führen ist.

Zur weiteren Prüfung der Frage, ob die relativ wenig (ca. 2fach) erhöhten "proAM-Immunreaktivitäts-Meßwerte" der früheren Messungen real gewesen waren, oder ob bei diesen Messungen möglicherweise Artefakte eine Rolle spielten, die auf den verwendeten kommerziellen RIA zurück zu führen waren, wurde ein weiterer Assay entwickelt, der auf dem sogenannten SPALT-Prinzip beruhte. Bei einem solchen Assay wird eine Kompetition zwischen einem festphasengebundenen (solid phase = SP) Kompetitor für den Analyten ("Antigen" = A) und dem Analyten um gemeinsame Bindungsstellen eines markierten Antikörpers, der sich in der Reaktionsflüssigkeit befindet, genutzt. Im vorliegenden Fall war der Antikörper mit einem Lumineszenz-Tracer (LT) markiert (vgl. Experimenteller Teil). Die Anwesenheit des Analyten, bzw. die Besetzung von Bindungsstellen des Antikörpers durch konkurrierende Bindungspartner aus der Probe, zeigt sich als Verminderung der Bindung des markierten Antikörpers an die Festphase.

Als festphasengebundener Kompetitor diente in dem beschriebenen SPALT-Assay das festphasengebundene Peptid (69-86: Peptidbereich SPCD19; SEQ ID NO:4), als Antikörper ein gegen dieses Peptid gebildeter und dieses Peptid erkennender markierter anti-SPCD19-Schaf-Antikörper (affinitätsgereinigt; vgl. Experimenteller Teil). Als Standard dienten Verdünnungen des Peptids SPCD19 in normalem Pferdeserum. Die Nachweisgrenze lag bei ca. 50 pmol/l. Bei den Bestimmungen wurden jeweils 100 µl Probe (bzw. Standard) und 100 µl Tracer bei 4°C in mit dem SPCD19-Peptid beschichteten Polysorb-Tubes über Nach inkubiert, wonach mit 4 x 1 ml Standardwaschlösung aus dem LUMItest® der Anmelderin gewaschen und dann in einem Luminometer vermessen wurde.

Bei einer Vermessung von Sepsisseren mit diesem SPALT-Assay wurde eine drastische Abgrenzung von Septikern gegen gesunde Normalpersonen gefunden. Bei einem Detektionslimit von ca. 1 ng/ml wurden für Seren von Septikern Werte gefunden, die im Mittel bei rund 19000 pg/ml lagen. Die deutliche Unterscheidung von Seren von Septikern und Gesunden war angesichts der eher geringfügigen Erhöhung bei den Vorversuchen unter Verwendung eines kommerziellen RIA (WO 00/22439) sehr überraschend.

Die klinischen Messungen mit dem genannten SPALT-Assay wurden ausgeweitet. Die Ergebnisse der ausgeweiteten Studie sind in Figur 1 graphisch zusammengeafßt, wobei auf die obige Erläuterung zu Figur 1 ausdrücklich verwiesen wird.

Die o.g. positiven Ergebnisse mit SPALT-Assays zeigten, dass (i) die in Sepsisseren gemessene "proAM-Immunreaktivität" zwar nicht auf die tatsächliche Anwesenheit von Proadrenomedullin zugeführt werden konnte, aber (ii) eine entsprechende Messung eines Analyten mit einer Aminosäuresequenz aus einem mittleren Abschnitt des pre-proAM, genauer des mid-proAM gemäß SEQ ID NO:3, geeignet war, Septiker klar von Normalpersonen zu unterscheiden.

Auf diesen Ergebnisse aufbauend wurde die Untersuchgun in zwei Richtungen vertieft:
1. Die tatsächlich in der Zirkulation auftretende pre-proAM Spezies sollte identifiziert und ggf. auf ihre Eignung als Biomarker für Routinemessungen untersucht werden.
2. Parallel dazu sollte vertieft werden, inwieweit die Messung dieser Spezies diagnostisch wertvolle Meßergebnisse liefert.

Die Ergebnisse sind nachfolgend unter Bezugnahme auf den experimentellen Teil noch näher beschrieben. Sie lassen sich wie folgt zusammen fassen:
1. In der Zirkulation (Serum, Plasma) findet sich in signifikant erhöher Konzentration, und einer gut reproduzierbaren Messbarkeit, ein Peptid, das die Aminosäuren 45-92 des pre-proAM enthält bzw. aus diesen besteht (SEQ ID NO:3) und das in dieser Anmeldung als mid-proAM bezeichnet wird.
2. Die Vermessung von Seren Kranker mit einem Assay, der spezifisch dieses mid-proAM mißt, liefert Meßergebnisse, die nicht nur eine klare Unterscheidung von Septikern und Normalpersonen ermöglichen, sondern die - in Verbindung mit klinischen Befunden - auch andere Erkrankungen erkennen lassen, die mit einer gesteigerten Bildung von AM verbunden sind, und zwar insbesondere Kardial- und Krebserkrankungen.

Das Verfahren betrifft somit insbesondere die Bestimmung des mid-proAM in der Zirkulation eines Patienten, und zwar insbesondere unter Verwendung von Plasmaproben.

Nachfolgend werden bestimmte allgemeine Aspekte bevorzugter Ausführungsformen der Erfindung noch näher erläutert, und es werden weitere ausgewählte Versuchsergebnisse näher erläutert.

Für die praktische Umsetzung der Erfindung wird ein Assayformat bevorzugt, bei dem mit markierten Antikörpern gearbeitet wird, z.B. ein Assay, der nach dem oben beschriebenen kompetitiven SPALT-Prinzip arbeitet (wobei jedoch auch andere Markierungen, z.B. radioaktive als SPART-Assay, eingesetzt werden können).

Besonders bevorzugt sind jedoch nicht-kompetitive Sandwichassays, z.B. der Art, wie er für die weitergehenden vertiefenden Untersuchungen verwendet wurde und nachfolgend genauer beschrieben wird.

Nicht-kompetitive Sandwich-Immunoassays (Zweiseiten-Immunoassays) haben gegenüber kompetitiven Immunoassays eine Reihe' von Vorteilen, zu denen gehört, daß sie besser als Festphasenassays (heterogene Assays) ausgelegt werden können, in der Handhabbarkeit robuster sein können, Meßergebnisse mit einer höheren Sensititivät liefern können und sich auch besser für eine Automatisierung und Serienmessung eignen. Außerdem können sie im Vergleich mit kompetitiven Immunoassays, die mit nur einem Typ von Antikörper arbeiten, auch zusätzliche Aussagen liefern, indem Sandwich-Immunoassays nur solche Moleküle bzw. Peptide erkennen, bei denen beide Bindungsstellen für die für die Sandwichbildung verwendeten Antikörper auf dem gleichen Molekül vorhanden sind.

Die Antikörper können grundsätzlich beliebige geeignete monoklonale und/oder polyklonale Antikörper sein, wobei jedoch derzeit affinätsgereinigte polyklonale Antikörper bevorzugt werden.

Besonders bevorzugt wird einer der Antikörper durch Immunisierung eines Tiers, insbesondere Schafs, mit einem Antigen erhalten, das eine synthetische Peptidsequenz enthält, die die Aminosäuren 69-86 des pre-proAM sowie einen zusätzlichen Cysteinrest am N-Terminus aufweist (SEQ ID NO:4). Der andere Antikörper kann z.B. entsprechend mit einem Antigen erhalten werden, das eine synthetische Peptidsequenz enthält, die die Aminosäuren 83-94 (Peptidbereich PSR13; SEQ ID NO:5) des pre-proAM mit einem zusätzlichen Cysteinrest am N-Terminus aufweist. Die unter Verwendung der genannten synthetischen Peptide, die gemeinsam einen lückenlosen midregionalen Abschnitt der proAM-Sequenz erfassen, erhaltenen Antikörper erkennen nur Bindungsstellen im Bereich des o.g. mid-proAM (Aminosäuren 45-92), genauer im Bereich der Aminosäuren 60-92 des pre-proAM.

Bei einer bevorzugten Ausführungsform wird das Verfahren als heterogener Sandwich-Immunoassay durchgeführt, bei dem einer der Antikörper an eine beliebige Festphase, beispielsweise die Wände beschichteter Teströhrchen (z.B. aus Polystyrol; "Coated Tubes"; CT) oder an Mikrotiterplatten, zum Beispiel aus Polystyrol, oder an Partikel, beispielsweise Magnetpartikel immobilisiert ist, während der andere Antikörper einen Rest trägt, der ein direkt nachweisbares Label darstellt oder eine selektive Verknüpfung mit einem Label ermöglicht und der Detektion der gebildeten Sandwich-Strukturen dient. Auch eine zeitlich verzögerte bzw. nachträgliche Immobilisierung unter Verwendung geeigneter Festphasen ist möglich.

Grundsätzlich können alle in Assays der beschriebenen Art verwendbaren Markierungstechniken angewandt werden, zu denen Markierungen mit Radioisotopen, Enzymen, Fluoreszenz-, Chemolumineszenz- oder Biolumineszenz-Labeln und direkt optisch detektierbaren Farbmarkierungen, wie beispielsweise Goldatomen und Farbstoffteilchen, wie sie insbesondere für sog. Point-of-Care (POC) oder Schnelltests verwendet werden, gehören. Die beiden Antikörper können auch im Falle heterogener Sandwich-Immunoassays Teile eines Nachweissystems der nachfolgend im Zusammenhang mit homogenen Assays beschriebenen Art aufweisen.

Es liegt somit im Rahmen der vorliegenden Erfindung, das erfindungsgemäße Verfahren auch als Schnelltest auszugestalten.

Das erfindungsgemäße Verfahren kann ferner als homogenes Verfahren ausgestaltet werden, bei dem die aus den beiden Antikörpern und dem nachzuweisenden mid-proAM gebildeten Sandwichkomplexe in der flüssigen Phase suspendiert bleiben. In einem solchen Fall ist es bevorzugt, beide Antikörper mit Teilen eines Nachweissystems zu markieren, das dann, wenn beide Antikörper in einen einzigen Sandwich integriert werden, eine Signalerzeugung oder Signalauslösung ermöglicht. Derartige Techniken sind insbesondere als Fluoreszenzverstärkungs- oder Fluoreszenzlöschungs-Nachweisverfahren ausgestaltbar. Ein besonderes bevorzugtes derartiges Verfahren betrifft die Verwendung von paarweise einzusetzenden Nachweisreagenzien, wie sie beispielsweise beschrieben sind in US-A-4 822 733, EP-B1-180 492 oder EP-B1-539 477 und dem darin zitierten Stand der Technik. Sie ermöglichen eine Messung, die selektiv nur Reaktionsprodukte erfaßt, die beide Markierungskomponenten in einem einzigen Immunkomplex enthalten, direkt in der Reaktionsmischung. Als Beispiel ist auf die unter den Marken TRACE® (Time Resolved Amplified Cryptate Emission) bzw. KRYPTOR® angebotene Technologie zu verweisen, die die Lehren der o.g. Anmeldungen umsetzt.

Es zeigte sich überraschenderweise, daß die erfindungsgemäße Bestimmung des mid-proAM (SEQ ID NO:3) hoch signifikante Meßergebnisse liefert. Diese Aussage gilt, wie nachfolgend gezeigt wird, nicht nur für die Sepsisdiagnostik, sondern auch für die Kardialdiagnostik und die Krebsdiagnostik.

Es wird davon ausgegangen, dass das erfindungsgemäße Bestimmungsverfahren besonders vorteilhaft auch im Rahmen einer sogenannten Multiparameterdiagnostik durchgeführt werden kann, und zwar sowohl auf dem Gebiet der Kardialdiagnostik als auch der Sepsis- und der Krebsdiagnostik. Weitere dabei bestimmte Parameter sind beispielsweise die Kardialparameter ANP, BNP, proANP oder proBNP oder Sepsisparameter, die z.B. aus der Gruppe ausgewählt sind, die besteht aus Anti-Gangliosid-Antikörpern, den Proteinen Procalcitonin, CA 125, CA 19-9, S100B, S100A-Proteinen, LASP-1, löslichen Cytokeratin-Fragmenten, insbesondere CYFRA 21, TPS und/oder löslichen Cytokeratin-1-Fragmenten (sCY1F), den Peptiden Inflammin und CHP, anderen Peptid-Prohormonen, der Glycin-N-Acyltransferase (GNAT), der Carbamoylphosphat Synthetase 1 (CPS 1) und dem C-reaktiven Protein (CRP) oder Fragmenten davon. Bei den genannten Multiparameter-Bestimmungen ist vorgesehen, die Messergebnisse für mehrere Parameter gleichzeitig oder parallel zu bestimmen und z.B. mit Hilfe eines Computerprogramms, das auch diagnostisch signifikante Parameterkorrelationen nutzt, auszuwerten.

Nachfolgend wird die Erfindung durch eine Beschreibung der Herstellung der bevorzugten Assay-Komponenten, der Durchführung einer bevorzugten Ausführungsform eines Assays vom Sandwich-Typ und der unter Verwendung eines solchen Assays erhaltenen Ergebnisse von mid-proAM-Bestimmungen in EDTA-Plasmen von Kontrollpersonen, und von Sepsis-, Herz- und Krebspatienten näher erläutert.

Ferner wird die Identifizierung des tatsächlich bestimmten, in der Zirkulation auftretenden proAM-Teilpeptids beschrieben.

### Experimenteller Teil

### Material und Methoden

### 1. Peptidsynthesen

Abgeleitet von der bekannten Aminosäuresequenz des humanen Präproadrenomedullin (SEQ ID NO:1) wurden ein erster Bereich (Pos. 69-86: Peptidbereich SPCD19; SEQ ID NO:4) und ein zweiter Bereich (Pos. 83-94: Peptidbereich PSR13; SEQ ID NO:5) ausgewählt. Jeweils ergänzt um einen N-terminalen Cystein-Rest wurden beide Bereiche nach Standardverfahren als lösliche Peptide chemisch synthetisiert, gereinigt, mittels Massenspektrometrie und Reversed Phase HPLC qualitätskontrolliert und in Aliquots lyophilisiert (Firma JERINI AG, Berlin, Deutschland). Die Aminosäuresequenzen der Peptide lauten: Außerdem wurde als Standard das gesamte mid-proAM (entsprechend Pos. 45-92; SEQ ID NO:3) synthetisiert:

### 2. Konjugation und Immunisierung

Mittels MBS (m-Maleimidobenzoyl-N-hydroxysuccinimid Ester) wurden die obigen Peptide SPCD19 bzw. PSR13 an das Trägerprotein KLH (Keyhole limpet hemocyanin) konjugiert (s. Arbeitsanleitung "NHS-Esters-Maleimide Crosslinkers" Firma PIERCE, Rockford, IL, USA). Mit diesen Konjugaten wurden Schafe nach folgendem Schema immunisiert: Jedes Schaf erhielt initial 100 µg Konjugat (Massenangabe bezogen auf den Peptid-Anteil des Konjugats) und anschließend 4-wöchentlich je 50 µg Konjugat (Massenangabe bezogen auf den Peptid-Anteil des Konjugats). Beginnend mit dem vierten Monat nach Beginn der Immunisierung wurden 4-wöchentlich je Schaf 700 ml Blut abgenommen und daraus durch Zentrifugation Antiserum gewonnen. Konjugationen, Immunisierungen und Gewinnung von Antiseren wurden von der Firma MicroPharm, Carmarthenshire, UK, durchgeführt.

### 3. Reinigung der Antikörper

In einem 1-Schritt Verfahren wurden aus den Antiseren, die beginnend mit dem vierten Monat nach der Immuniserung gewonnen worden waren, die Peptid-spezifischen Antikörper wie folgt präpariert.

Dazu wurden zunächst die o.g. Peptide SPCD19 und PSR13 an SulfoLink Gel gekoppelt (s. Arbeitsanleitung "SulfoLink Kit" Firma PIERCE, Rockford, IL, USA). Dabei wurden zur Kopplung je 5 mg Peptid pro 5 ml Gel angeboten.

Die Affinitätsreinigung von Peptid-spezifischen Antikörpern aus Schaf-Antiseren gegen beide Peptide wurde wie folgt durchgeführt:

Die Peptid-Säulen wurden zunächst drei mal im Wechsel mit je 10 ml Elutionspuffer (50 mM Citronensäure, pH 2.2) und Bindungspuffer (100 mM Natriumphosphat, 0.1% Tween, pH 6.8) gewaschen. 100 ml der Schaf-Antiseren wurden über 0,2 µm filtriert und mit dem vorhandenen Säulenmaterial versetzt. Dazu wurde das Gel quantitativ mit 10 ml Bindungspuffer aus der Säule gespült. Die Inkubation erfolgte über Nacht bei Raumtemperatur unter Schwenken. Die Ansätze wurden quantitativ in Leersäulen (NAP 25, Pharmacia, entleert) überführt. Die Durchläufe wurden verworfen. Anschließend wurde mit 250 ml Bindungspuffer proteinfrei (Proteingehalt des Wascheluats < 0,02 A280 nm) gewaschen. Auf die gewaschene Säulen wurde Elutionspuffer gegeben, und es wurden Fraktionen ä 1 ml gesammelt. Von jeder Fraktion wurde der Proteingehalt mittels der BCA-Methode (s. Arbeitsanleitung Firma PIERCE, Rockford, IL, USA) bestimmt. Fraktionen mit Proteinkonzentrationen > 0.8 mg/ml wurden gepoolt. Nach einer Proteinbestimmung der Pools mittels der BCA-Methode ergaben sich Ausbeuten von 49 mg für den anti-SPCD19 Antikörper (affinitätsgereinigt; polyklonal) und 60 mg für den anti-PSR13 Antikörper (affinitätsgereinigt; polykonal).

### 4. Markierung

Über eine NAP-5 Gelfiltrationssäule (Pharmacia) wurden 500 µl des gereinigten anti-SPCD19 Antikörpers (s.o.) in 1 ml 100 mM Kalium-Phosphatpuffer (pH 8,0) nach Arbeitsanleitung umgepuffert. Die Proteinkonzentationsbestimmung der Antikörperlösung ergab einen Wert von 1,5 mg/ml.

Zur Chemilumineszenzmarkierung des Antikörpers wurden 67 µl der Antikörperlösung mit 10 µl MA70-Akridinium-NHS-Ester (1 mg/ml; Firma HOECHST Behring) versetzt und 15 Minuten bei Raumtemperatur inkubiert. Dann wurden 423 µl 1 M Glycin zugesetzt und weitere 10 Minuten inkubiert. Anschließend wurde der Markierungsansatz über eine NAP-5 Gelfiltrationssäule (Pharmacia) in 1 ml Laufmittel A (50 mM Kaliumphosphat, 100 mM NaCl, pH 7.4) nach Arbeitsanleitung umgepuffert und dabei von niedermolekularen Bestandteilen befreit. Zur Abtrennung letzter Reste nicht an Antikörper gebundenen Labels wurde eine Gelfiltrations-HPLC durchgeführt (Säule: Waters Protein Pak SW300). Die Probe wurde aufgetragen und bei einer Flußrate von 1 ml/min mit Laufmittel A chromatographiert. Mit einem Durchflußphotometer wurden die Wellenlängen 280 nm und 368 nm gemessen. Das Absorptionsverhältnis 368 nm/280 nm als Maß für den Markierungsgrad des Antikörpers betrug am Peak 0.10. Die monomeren Antikörper enthaltenden Fraktionen (Retentionszeit 8-10 min) wurden gesammelt und in 3 ml 100 mM Natriumphosphat, 150 mM NaCl, 5% Bovines Serum Albumin, 0.1% Natrium Azid, pH 7.4, aufgenommen.

Der markierte Antikörper wurde einerseits, wie nachfolgend genauer beschrieben wird, in einem Sandwich-Immunoassay verwendet, andererseits jedoch auch in dem bereits beschriebenen SPALT-Assay.

### 5. Kopplung

Zur Schaffung der Festphase eines Sandwich-Immunoassays wurden bestrahlte 5 ml Polystyrolröhrchen (Firma Greiner) mit gereinigtem anti-PSR13 Antikörper wie folgt beschichtet: Der Antikörper wurde in 50 mM Tris, 100 mM NaCl, pH 7,8 zu einer Konzentration von 6.6 µg/ml verdünnt. In jedes Röhrchen wurden 300 µl dieser Lösung pipettiert. Die Röhrchen wurden 20 Stunden bei 22°C inkubiert. Die Lösung wurde abgesaugt. Dann wurde jedes Röhrchen mit 4.2 ml 10 mM Natriumphosphat, 2% Karion FP, 0.3% Bovines Serum Albumin, pH 6.5 befüllt. Nach 20 Stunden wurde die Lösung abgesaugt. Schließlich wurden die Röhrchen in einem Vakuumtrockner getrocknet.

Die beschriebenen Markierungs- und Immobilisierungsprozeduren wurden außerdem auf im wesentlichen gleiche Weise mit dem jeweils anderen Antikörper durchgeführt, wobei ein "inverser" Sandwichassay erhalten wurde. Bestimmungen, die analog zu den nachfolgend beschriebenen Bestimmungen unter Verwendung eines solchen "invers" markierten/immobilisierten Immunoassays durchgeführt wurden, lieferten im wesentlichen identische Ergebnisse und werden daher nicht extra beschrieben.

### 6. Durchführung und Auswertung des Sandwich-Immunoassays

Es wurde ein Assaypuffer folgender Zusammensetzung hergestellt:
100 mM Natriumphosphat, 150 mM NaCl, 5% Bovines Serum Albumin, 0.1% unspez. Schaf IgG, 0.1% Natrium Azid, pH 7.4

Als Standardmaterial diente ein chemisch synthetisiertes mid-proAM (SEQ ID NO:3). Dieses Peptid wurde seriell in Pferdenormalserum (Firma SIGMA) verdünnt. Den so hergestellten Standards wurden Konzentrationen gemäß der Einwaage an Peptid zugeschrieben.

Meßproben waren EDTA-Plasmen von augenscheinlich Gesunden, von Patienten mit Sepsis und von Patienten mit Herz- und mit Krebserkrankungen.

In die Teströhrchen wurden 10 µl Standards bzw. Proben sowie 200 µl Assaypuffer, enthaltend 1 Million RLU (relative light units) des MA70-markierten anti-SPCD19 Antikörpers, pipettiert. Es wurde zwei Stunden bei 22°C unter Schütteln inkubiert. Dann wurde 4 x mit je 1 ml Waschlösung (0.1% Tween 20) pro Röhrchen gewaschen, abtropfen gelassen und die am Röhrchen gebundene Chemilumineszenz in einem Luminometer (Firma BERTHOLD, LB952T; Basisreagenzien BRAHMS AG) vermessen.

Unter Verwendung der Software MultiCalc (Spline Fit) wurden die midregionalen Proadrenomedullin-Konzentrationen der Proben an der Standardkurve abgelesen. Die Ergebnisse sind in Figur 2 graphisch zusammengefaßt.

### 7. Durchführung und Auswertung des SPALT-Immunoassays

Als festphasengebundener Kompetitor diente in dem beschriebenen SPALT-Assay das festphasengebundene Peptid SPCD19 (Peptidbereich 69-86; SEQ ID NO:4), das an die Wände von Polysorb-Tubes gebunden worden war. Als Antikörper wurde der wie oben unter 1. bis 4. beschrieben erhaltene markierte anti-SPCD19-Schaf-Antikörper (affinitätsgereinigt) verwendet. Als Standard dienten Verdünnungen des Peptids SPCD19 in normalem Pferdeserum.

Bei den Bestimmungen wurden jeweils 100 µl Probe (bzw. Standard) und 100 µl Tracer bei 4°C in den mit dem SPCD19-Peptid beschichteten Polysorb-Tubes über Nacht inkubiert, wonach mit 4 x 1 ml Standardwaschlösung aus dem LUMItest® der Anmelderin gewaschen und dann im Luminometer vermessen wurde.

Die Ergebnisse einer mit diesem Assay erhaltenen Meßreihe sind in Figur 1 dargestellt.

### 8. Identifikation des in den beschriebenen Assays gemessenen Analyten

Zur Anreicherung des Analyten, der von dem in den o.g. Assays verwendeten Antikörper erkannt wird, wurden drei individuelle Sepsisplasmen direkt analytisch über eine C18 Reversed Phase HPLC fraktioniert, wobei mittels eines linearen Acetonitril-Gradienten eluiert wurde. Es wurden 1 ml-Fraktionen gesammelt und eingetrocknet. Die Fraktionen wurden in Assaypuffer aufgenommen, und es wurde die SPCD19-Immunreaktivität der einzelnen Fraktionen bestimmt. Dazu wurde ein anti-SPCD19-Antikörper (vgl. oben unter 3.) an den Wänden eines Polysorb-Tubes immobilisiert, und die Kompetition von Probe (Fraktion) und lumineszenzmarkiertem SPCD19 um diesen Antikörper wurde bestimmt.

Bei einer solchen Analyse zeigte sich, dass bei allen Sepsisplasmen die größte Immunreaktivität in der gleichen Fraktion (Fraktion 22) zu finden war.

Zur weiteren Identifizierung des gemessenen Analyten wurden 7 Sepsisseren á ca. 3 ml gepoolt (Endvolumen 22 ml). Unter Verwendung einer Carbolink-Säule mit einem anti-SPCD19-Antikörper wurden die gepoolten Seren einer Affinitätsreinigung unterzogen, und das saure Eluat wurde wie oben über eine C18 Reversed Phase HPLC fraktioniert. Die Fraktion 22 wurde getrocknet und massenspektrometrisch untersucht.

Bei einer direkten massenspektrometrischen Analyse wurde als Molmasse des isolierten Analyten ein Wert von ca. 5146 Dalton ermittelt. Dieser Wert entspricht der Molmasse eines proAM-Fragments, das die Aminosäuren der Positionen 45-92, d.h. des mid-proAM, enthält (der theoretische Wert beträgt, unter der Annahme, dass die beiden vorhandenen Methionin-Reste oxidert sind, 5146,72 Dalton).

Bei einer MALDI-TOF-Analyse des tryptischen Verdaus der isolierten Fraktion 22 wurden als monoisotopische Massen (M + H⁺) u.a. Peptid-Fragmente identifiziert, die den Aminosäuren der Positionen 79-89, 75-89, 61-74 und 61-78 des pre-proAM entsprechen. Die Molmassedaten und die massenspektrometrische Analyse des tryptischen Abbaus beweisen gemeinsam, dass das in der isolierten Fraktion enthaltene Peptid das als mid-proAM (45-92) bezeichnete Peptid (SEQ ID NO:3) ist. Seine Entstehung kann durch eine proteolytische Prozessierung des ursprünglichen pre-proAM-Translationsprodukts durch Signalpeptidase, Prohormonconvertase (Spaltung zwischen basischen Aminosäuren) und Amino- und Carboxypeptidase (Abspaltung der basischen Aminosäuren) erklärt werden (vgl. das analoge Schema für den Procalcitonin-Abbau in (20)).

### 9. Stabilitätsuntersuchung

Zur Prüfung der Frage, ob bei einer Messung des mid-proAM mit Problemen aufgrund einer unzureichenden Stabilität des mid-proAM in einer Probe bzw. Messlösung gerechnet werden muß, wurden 20 Sepsis-Seren jeweils frisch und nach einer 3 tägigen Lagerung bei Raumtemperatur vermessen. Die Ergebnisse sind in der nachfolgenden Tabelle zusammengefaßt. Sie zeigen, dass nach der 3tägigen Lagerung die Immunreaktivität nahezu unverändert war. Diese nachgewiesene Stabilität des mid-proAM ist unter Handhabungsgesichtspunkten für die Diagnostik ein großer Vorteil.

**Tabelle 1**

| Patient # | mid-proAM [nmol/l] Tag = 0 | mid-proAM [nmol/l] Tag = 3 | Änderung |
|---|---|---|---|
| 1 | 6,2 | 6,1 | 98,8% |
| 2 | 3,3 | 3,2 | 98,1% |
| 3 | 2,2 | 2,1 | 97,0% |
| 4 | 1,6 | 1,5 | 95,4% |
| 5 | 1,1 | 1,0 | 92,7% |
| 6 | 1,3 | 1,2 | 95,7% |
| 7 | 1,9 | 2,1 | 109,6% |
| 8 | 2,6 | 2,7 | 102,8% |
| 9 | 2,8 | 2,7 | 96,4% |
| 10 | 3,1 | 3,1 | 99,9% |
| 11 | 4,6 | 4,9 | 106,3% |
| 12 | 5,8 | 5,9 | 102,1% |
| 13 | 3,6 | 3,4 | 95,2% |
| 14 | 4,2 | 4,6 | 110,7% |
| 15 | 3,0 | 2,4 | 80,0% |
| 16 | 1,2 | 1,3 | 105,5% |
| 17 | 1,5 | 1,5 | 102,2% |
| 18 | 1,7 | 1,8 | 103,4% |
| 19 | 2,0 | 1,8 | 89,5% |
| 20 | 2,1 | 2,0 | 94,1% |
| Mittelwert = 98,8% | | | |

Zusammenfassend kann gesagt werden, dass eine Bestimmung von mid-proAM, z.B. unter Verwendung eines SPCD19-Antikörpers, gegenüber einer Bestimmung von z.B. AM zahlreiche Vorteile aufweist:

Eine Bestimmung des mid-proAM unterliegt keinen bekannten Einschränkungen aufgrund der Existenz eines Bindeproteins, einer Fragmentierung und einer schwachen Konzentrationsdynamik.

Der Analyt mid-proAM weist ferner eine gute Stabilität auf, d.h. einen sehr geringen Verlust an Immunreaktivität bei Lagerung bei Raumtemperatur, was für diagnostische RoutineBestimmungen einen großen praktischen Vorteil darstellt.

Es wird eine extrem günstige Dynamik beobachtet, und es nicht davon auszugehen, dass diese spezifisch für Sepsis ist.

Es ist daher davon auszugehen, dass eine Messung von mid-proAM generell bei allen Krankheitsbildern, für die AM-Konzentrationserhöhungen beschrieben werden, Vorteile aufweisen kann, wobei derzeit insbesondere eine Bestimmung im Rahmen der Sepsis-, Kardial- und Krebsdiagnostik vorteilhaft erscheint.

### Literaturliste

1. K. Ehlenz et al.: "High levels of circulating adrenomedullin in severe illness: Correlation with C-reactive protein and evidence against the adrenal medulla as site of origin", Exp. Clin. Endocrinol. Diabetes (1997) 105, 156-162
2. Tanenao Eto: "A review of the biological properties and clinical implications of adrenomedullin and proadrenomedullin N-terminal 20 peptide (PAMP), hypotensive and vasodilating peptides", Peptides (2001) 22, 1693-1711
3. Joy Patricia Hinson et al.: "Adrenomedullin, a multifunctional regulatory peptide", Endocr. Rev. (2000) 21(2), 138-167
4. Yukio Hirata et al.: "Increased circulating adrenomedullin, a novel vasodilatory peptide, in sepsis", J. Clin. Endocrinol. Metab. (1996) Vol. 81, Nr. 4, 1449-1453
5. K. Kitamura et al.: "The intermediate form of glycineextended adrenomedullin is the major circulating molecular form in human plasma", Biochem. Biophys. Res. Commun. (1998) 244(2), 551-555
6. Kazuo Kitamura et al.: "Adrenomedullin (11-26): a novel endogenous hypertensive peptide isolated from bovine adrenal medulla", Peptides (2001) 22, 1713-1718
7. M. Kohno et al.: "Plasma adrenomedullin concentrations in essential hypertension", Hypertension (1996) 27(1), 102-107
8. K. Kuwasako et al.: "Purification and characterization of PAMP-12 (PAMP-20) in porcine adrenal medulla as a major endogenous biologically active peptide", FEBS Lett (1997) 414(1), 105-110
9. K. Kuwasako et al.: "Increased plasma proadrenomedullin N-terminal 20 peptide in patients with essential hypertension", Ann. Clin. Biochem. (1999) 36 (Pt 5), 622-628
10. Lynley K. Lewis et al.: "Adrenomedullin(1-52) measured in human plasma by radioimmunoassay: plasma concentration, adsorption, and storage", Clin. Chem. (1998) 44:3, 571-577
11. Rubén Pío et al.: "Complement factor H is a serum-binding protein for adrenomedullin, and the resulting complex modulates the bioactivities of both partners", J. Biol. Chem. (2001) Vol. 276, Nr. 15, 12292-12300
12. Kazuhiro Takahashi: "Adrenomedullin: from a pheochromocytoma to the eyes", Peptides (2001) 22, 1691
13. Yoshio Tomoda et al.: "Regulation of adrenomedullin secretion from cultured cells", Peptides (2001) 22, 1783-1794
14. T. Tsuruda et al.: "Secretion of proadrenomedullin N-terminal 20 peptide from cultured neonatal rat cardiac cells", Life Sci. (2001) 69(2), 239-245
15. Shiro Ueda et al.: "Increased plasma levels of adrenomedullin in patients with systemic inflammatory response syndrome", Am. J. Respir. Crit. Care Med. (1999) Vol. 160, 132-136
16. Ping Wang: "Adrenomedullin and cardiovascular responses in sepsis", Peptides (2001) 22, 1835-1840
17. H. Washimine et al.: "Plasma concentration of human adrenomedullin in patients on hemodialysis", Clin. Nephrol. (1995) 44(6), 389-393
18. K. Kitamura et al.: "Adrenomedullin: A Novel Hypotensive Peptide Isolated From Human Pheochromocytoma"; Biochem.Biophys.Res.Commun. 192:553-560 (1993)
19. K. Kitamura et al.: "Cloning And Characterization of cDNA Encoding a Precursor for Human Adrenomedullin"; Biochem.Biophys.Res.Commun. 194:720-725 (1993)
20. Meisner M., (2000) "Procalcitonin", Georg Thieme Verlag, ISBN 3-13-105473-5, S.22

## Patentansprüche

1. In vitro-Verfahren zur Bestimmung von Adrenomedullin-Immunreaktivität in biologischen Flüssigkeiten zu diagnostischen Zwecken, **dadurch gekennzeichnet, dass** man das midregionale Teilpeptid (mid-proAM; SEQ ID NO:3) des Proadrenomedullins, das die Aminosäuren (45-92) des vollständigen Präproadrenomedullins (pre-proAM; SEQ ID NO:1) umfaßt, mißt.

2. Verfahren nach Anspruch 1, **dadurch gekennzeichnet, dass** man das mid-proAM in den biologischen Flüssigkeiten mittels eines Immunoassays mißt, der mit mindestens einem markierten Antikörper, der spezifisch eine Sequenz des mid-proAM erkennt, arbeitet.

3. Verfahren nach Anspruch 2, **dadurch gekennzeichnet, dass** der Immunoassay ein Assay mit einem festphasengebundenen Kompetitor für den Analyten und einem markierten Antikörper (SPALT-Assay) oder ein Sandwichassay (Zweiseiten-Immunoassay) ist, bei dem mindestens zwei Antikörper verwendet werden, die spezifisch an unterschiedliche Teilsequenzen des mid-proAM (SEQ ID NO:3) binden.

4. Verfahren nach einem der Ansprüche 1 bis 3, **dadurch gekennzeichnet, dass** zirkulierendes mid-proAM (SEQ ID NO:3) bestimmt wird und die biologische Flüssigkeit ein Plasma ist.

5. Verfahren nach Anspruch 3, **dadurch gekennzeichnet, dass** beide Antikörper an einen Bereich des mid-proAM binden, der sich von der Aminosäure 60 bis zur Aminosäure 94 des pre-proAM erstreckt.

6. Verfahren nach einem der Ansprüche 1 bis 5, **dadurch gekennzeichnet, dass** der/die Antikörper monoklonale und/oder polyklonale Antikörper sind.

7. Verfahren nach einem der Ansprüche 1 bis 6, **dadurch gekennzeichnet, dass** beide Antikörper affinitätsgereinigte polyklonale Antikörper sind.

8. Verfahren nach einem der Ansprüche 1 bis 7, **dadurch gekennzeichnet, dass** einer der Antikörper durch Immunisierung eines Tiers mit einem Antigen erhalten wird, das eine synthetischen Peptidsequenz enthält, die die Aminosäuren 69-86 des pre-proAM (SEQ ID NO:4) umfaßt, und der andere der Antikörper durch Immunisierung mit einem Antigen erhalten wird, das eine synthetische Peptidsequenz enthält, die die Aminosäuren 83-94 des pre-proAM (SEQ ID NO:5) umfaßt.

9. Verfahren nach einem der Ansprüche 1 bis 8, **dadurch gekennzeichnet, dass** einer der Antikörper markiert ist und der andere Antikörper an eine Festphase gebunden ist oder selektiv an eine Festphase gebunden werden kann.

10. Verfahren nach einem der Ansprüche 1 bis 8, **dadurch gekennzeichnet, dass** sowohl der erste als auch der zweite Antikörer in der flüssigen Reaktionsmischung dispergiert vorliegen und daß an den ersten Antikörper eine erste Markierungskomponente gebunden ist, die Teil eines auf Fluoreszenz- oder Chemilumineszenz-Löschung oder -Verstärkung beruhenden Markierungssystems ist, und daß die zweite Markierungskomponente dieses Markierungssystems an den zweiten Antikörper gebunden ist, so dass nach Bindung beider Antikörper an das nachzuweisende mid-proAM ein messbares Signal erzeugt wird, das eine Detektion der gebildeten Sandwichkomplexe in der Messlösung ermöglicht.

11. Verfahren nach Anspruch 10, **dadurch gekennzeichnet, dass** das Markierungssystem Seltenerdkrypate oder -chelate in Kombination mit einem Fluoreszenz- oder Chemilumineszenz-Farbstoff, insbesondere von Cyanintyp, umfaßt.

12. Verfahren nach einem der Ansprüche 1 bis 11, **dadurch gekennzeichnet, dass** es zur Diagnose, für die Bestimmung der Schweregrads und Prognostik sowie zur verlaufsbegleitenden Therapiekontrolle von Sepsis angewandt wird.

13. Verfahren nach Anspruch 12, **dadurch gekennzeichnet, dass** es im Rahmen einer Multiparameter-Bestimmung durchgeführt wird, bei der gleichzeitig mindestens ein weiterer für die Sepsisdiagnostik relevanter Parameter bestimmt wird.

14. Verfahren nach Anspruch 13, **dadurch gekennzeichnet, dass** der oder die weitere(n) für die Sepsisdiagnostik relevante(n) Parameter aus der Gruppe ausgewählt sind, die besteht aus Anti-Gangliosid-Antikörpern, den Proteinen Procalcitonin, CA 125, CA 19-9, S100B, S100A-Proteinen, LASP-1, löslichen Cytokeratin-Fragmenten, insbesondere CYFRA 21, TPS und/oder löslichen Cytokeratin-1-Fragmenten (sCY1F), den Peptiden Inflammin und CHP, anderen Peptid-Prohormonen, der Glycin-N-Acyltransferase (GNAT), der Carbamoylphosphat Synthetase 1 (CPS 1) und dem C-reaktiven Protein (CRP) oder Fragmenten davon.

15. Verfahren nach einem der Ansprüche 1 bis 11, **dadurch gekennzeichnet, dass** es auf dem Gebiet der Kardialdiagnostik angewandt wird.

16. Verfahren nach Anspruch 15, **dadurch gekennzeichnet, dass** es im Rahmen einer Multiparameter-Bestimmung durchgeführt wird, bei der gleichzeitig weitere für die Kardialdiagnostik relevante Parameter bestimmt werden.

17. Verfahren nach einem der Ansprüche 1 bis 11, **dadurch gekennzeichnet, dass** es auf dem Gebiet der Krebsdiagnostik angewandt wird.

18. Verfahren nach Anspruch 17, **dadurch gekennzeichnet, dass** es im Rahmen einer Multiparameter-Bestimmung durchgeführt wird, bei der gleichzeitig weitere für die Krebsdiagnostik relevante Parameter bestimmt werden.

## Claims

1. *In vitro* method for the determination of adrenomedullin immunoreactivity in biological fluids for diagnostic purposes, **characterized in that** the midregional partial peptide (mid-proAM; SEQ ID NO:3) of proadrenomedullin which comprises the amino acids (45-92) of the complete preproadrenomedullin (pre-proAM; SEQ ID NO:1) is measured.

2. Method according to Claim 1, **characterized in that** the mid-proAM in the biological fluids is measured by means of an immunoassay which operates with at least one labelled antibody which specifically recognizes a sequence of mid-proAM.

3. Method according to Claim 2, **characterized in that** the immunoassay is an assay with a solid phase-bound competitor for the analyte and a labelled antibody (SPALT assay) or a sandwich assay (two-sided immunoassay), in which at least two antibodies which specifically bind to different partial sequences of mid-proAM (SEQ ID NO:3) are used.

4. Method according to any of Claims 1 to 3, **characterized in that** circulating mid-proAM (SEQ ID NO:3) is determined and the biological fluid is a plasma.

5. Method according to Claim 3, **characterized in that** both antibodies bind to a region of mid-proAM which extends from the amino acid 60 to the amino acid 94 of the pre-proAM.

6. Method according to any of Claims 1 to 5, **characterized in that** the antibody/antibodies is/are monoclonal and/or polyclonal.

7. Method according to any of Claims 1 to 6, **characterized in that** both antibodies are affinity-purified polyclonal antibodies.

8. Method according to any of Claims 1 to 7, **characterized in that** one of the antibodies is obtained by immunization of an animal with an antigen which contains a synthetic peptide sequence which comprises the amino acids 69-86 of pre-proAM (SEQ ID NO:4), and the other of the antibodies is obtained by immunization with an antigen which contains a synthetic peptide sequence which comprises the amino acids 83-94 of pre-proAM (SEQ ID NO:5).

9. Method according to any of Claims 1 to 8, **characterized in that** one of the antibodies is labelled and the other antibody is bound to a solid phase or can be bound selectively to a solid phase.

10. Method according to any of Claims 1 to 8, **characterized in that** both the first and the second antibody are present dispersed in the liquid reaction mixture and that a first labelling component which is part of a labelling system based on fluorescence or chemiluminescence extinction or amplification is bound to the first antibody, and that the second labelling component of this labelling system is bound to the second antibody so that, after binding of both antibodies to the mid-proAM to be detected, a measurable signal which permits detection of the resulting sandwich complexes in the measuring solution is generated.

11. Method according to Claim 10, **characterized in that** the labelling system comprises rare earth cryptates or chelates in combination with a fluorescent or chemiluminescent dye, in particular of the cyanine type.

12. Method according to any of Claims 1 to 11, **characterized in that** it is used for diagnosis, for determination of the severity and prognosis and for therapy control accompanying the course of sepsis.

13. Method according to Claim 12, **characterized in that** it is carried out as part of a multiparameter determination in which at least one further parameter relevant for sepsis diagnosis is determined at the same time.

14. Method according to Claim 13, **characterized in that** the further parameter or parameters relevant for sepsis diagnosis is or are selected from the group consisting of anti-ganglioside antibodies, the proteins procalcitonin, CA 125, CA 19-9, S100B, S100A proteins, LASP-1, soluble cytokeratin fragments, in particular CYFRA 21, TPS and/or soluble cytokeratin-1 fragments (sCY1F), the peptides inflammin and CHP, other peptide prohormones, glycine-N-acyltransferase (GNAT), the carbamoylphosphate synthetase 1 (CPS 1) and the C-reactive protein (CRP) or fragments thereof.

15. Method according to any of Claims 1 to 11, **characterized in that** it is used in the area of cardiac diagnosis.

16. Method according to Claim 15, **characterized in that** it is carried out in the course of a multiparameter determination in which further parameters relevant for cardiac diagnosis are determined at the same time.

17. Method according to any of Claims 1 to 11, **characterized in that** it is used in the area of cancer diagnosis.

18. Method according to Claim 17, **characterized in that** it is carried out in the course of a multiparameter determination in which further parameters relevant for cancer diagnosis are determined at the same time.

## Revendications

1. Procédé de détermination in vitro de l'immunoréactivité à l'adrénomédulline dans des liquides biologiques dans un but de diagnostic, **caractérisé en ce que** l'on mesure le peptide partiel de la région médiane (mid-proAM; SEQ ID NO:3) de la proadrénomédulline, qui comprend les acides aminés (45-92) de la préproadrénomédulline complète (pré-proAM; SED ID NO:1).

2. Procédé selon la revendication 1, **caractérisé en ce que** l'on mesure la mid-proAM dans les liquides biologiques au moyen d'une immunodétermination qui fonctionne avec au moins un anticorps marqué qui reconnaît spécifiquement une séquence de mid-proAM.

3. Procédé selon la revendication 2, **caractérisé en ce que** l'immunodétermination est une détermination au moyen d'un compétiteur des analytes qui est lié à une phase solide et d'un anticorps marqué (test SPALT) ou est un test par sandwich (test immunologique bilatéral), dans lequel on utilise au moins deux anticorps qui se lient spécifiquement à différentes séquences partielles de la mid-proAM (SEQ ID NO:3).

4. Procédé selon l'une des revendications 1 à 3, **caractérisé en ce que** l'on détermine la mid-proAM en circulation (SEQ ID NO:3) et **en ce que** le liquide biologique est un plasma.

5. Procédé selon la revendication 3, **caractérisé en ce que** les deux anticorps se lient à une zone de la mid-proAM qui s'étend de l'acide aminé 60 jusqu'à l'acide aminé 94 de la pré-proAM.

6. Procédé selon l'une des revendications 1 à 5, **caractérisé en ce que** le ou les anticorps sont des anticorps monoclonaux et/ou polyclonaux.

7. Procédé selon l'une des revendications 1 à 6, **caractérisé en ce que** les deux anticorps sont des anticorps polyclonaux purifiés par affinité.

8. Procédé selon l'une des revendications 1 à 7, **caractérisé en ce que** l'un des anticorps est obtenu par immunisation d'un animal au moyen d'un antigène qui contient une séquence peptidique de synthèse qui comprend les acides aminés 69 à 86 de la pré-proAM (SEQ ID NO:4), l'autre anticorps étant obtenu par immunisation au moyen d'un antigène qui comprend une séquence peptidique de synthèse qui comprend les acides aminés 83 à 94 de la pré-proAM (SEQ ID NO:5).

9. Procédé selon l'une des revendications 1 à 8, **caractérisé en ce que** l'un des anticorps est marqué et l'autre anticorps est lié à une phase solide ou peut être lié sélectivement à une phase solide.

10. Procédé selon l'une des revendications 1 à 8, **caractérisé en ce que** tant le premier que le deuxième anticorps sont en dispersion dans le mélange réactionnel liquide et **en ce qu'**un premier composant de marquage est lié au premier anticorps et fait partie d'un système de marquage basé sur l'atténuation ou sur le renforcement de la fluorescence ou de la chimioluminescence et **en ce que** le deuxième composant de marquage de ce système de marquage est lié au deuxième anticorps, de telle sorte qu'une fois que les deux anticorps se sont liés à la mid-proAM à déceler, un signal mesurable est créé et permet de détecter le complexe sandwich formé dans la solution de mesure.

11. Procédé selon la revendication 10, **caractérisé en ce que** le système de marquage comprend des cryptates de terre rare ou des chélates de terre rare en combinaison avec un colorant fluorescent ou un colorant chimioluminescent, en particulier du type cyanine.

12. Procédé selon l'une des revendications 1 à 11, **caractérisé en ce qu'**il est utilisé pour diagnostiquer la septicémie, en déterminer le degré de gravité, faire des pronostics et contrôler la thérapie qui accompagne l'évolution de la maladie.

13. Procédé selon la revendication 12, **caractérisé en ce qu'**il est exécuté dans le cadre d'une détermination de plusieurs paramètres, dans laquelle on détermine en même temps au moins un autre paramètre pertinent pour le diagnostic de la septicémie.

14. Procédé selon la revendication 13, **caractérisé en ce que** le ou les autres paramètres pertinents pour le diagnostic de la septicémie sont sélectionnés dans le groupe constitué des anticorps antigangliosides, des protéines procalcitonine, CA 125, CA 19-9, S100B et S100A, des fragments solubles de cytokératine et en particulier des fragments solubles de CYFRA 21, de TPS et/ou de cytokératine-1 (sCYIF), des peptides inflammine et CHP, d'autres prohormones peptidiques, de la glycine-N-acyltransférase (GNAT), de la phosphate carbamoyle synthétase 1 de (CPS 1) et de la protéine C-réactive (CRP) ou de fragments de celle-ci.

15. Procédé selon l'une des revendications 1 à 11, **caractérisé en ce qu'**il est utilisé en diagnostic cardiologique.

16. Procédé selon la revendication 15, **caractérisé en ce qu'**il est exécuté dans le cadre de la détermination de plusieurs paramètres dans laquelle on détermine en même temps d'autres paramètres pertinents pour le diagnostic cardiologique.

17. Procédé selon l'un des revendications 1 à 11, **caractérisé en ce qu'**on l'utilise dans le domaine du diagnostic du cancer.

18. Procédé selon la revendication 17, **caractérisé en ce qu'**il est exécuté dans le cadre de la détermination de plusieurs paramètres dans laquelle on détermine en même temps d'autres paramètres pertinents pour le diagnostic du cancer.


### SEQUENCE LISTING

<110> B.R.A.H.M.S Aktiengesellschaft
<120> Bestimmung eines midregionalen Proadrenomedullin-Teilpeptids in biologischen Proben zu diagnostischen Zwecken sowie Immunoassays für die Durchführung einer solchen Bestimmung
<130> 3723 PCT AS
<140>
   <141>
<150> DE 10316583.5
   <151> 2003-04-10
<160> 5
<170> PatentIn Ver. 2.1
<210> 1
   <211> 185
   <212> PRT
   <213> Homo sapiens
<400> 1
   Met Lys Leu Val Ser Val Ala Leu Met Tyr Leu Gly Ser Leu Ala Phe
    1               5                  10                  15
   Leu Gly Ala Asp Thr Ala Arg Leu Asp Val Ala Ser Glu Phe Arg Lys
               20                  25                  30
   Lys Trp Asn Lys Trp Ala Leu Ser Arg Gly Lys Arg Glu Leu Arg Met
           35                  40                  45
   Ser Ser Ser Tyr Pro Thr Gly Leu Ala Asp Val Lys Ala Gly Pro Ala
       50                  55                  60
   Gln Thr Leu Ile Arg Pro Gln Asp Met Lys Gly Ala Ser Arg Ser Pro
   65                  70                  75                  80
   Glu Asp Ser Ser Pro Asp Ala Ala Arg Ile Arg Val Lys Arg Tyr Arg
                   85                  90                  95
   Gln Ser Met Asn Asn Phe Gln Gly Leu Arg Ser Phe Gly Cys Arg Phe
               100                 105                 110
   Gly Thr Cys Thr Val Gln Lys Leu Ala His Gln Ile Tyr Gln Phe Thr
           115                 120                 125
   Asp Lys Asp Lys Asp Asn Val Ala Pro Arg Ser Lys Ile Ser Pro Gln
       130                 135                 140
   Gly Tyr Gly Arg Arg Arg Arg Arg Ser Leu Pro Glu Ala Gly Pro Gly
   145                 150                 155                 160
   Arg Thr Leu Val Ser Ser Lys Pro Gln Ala His Gly Ala Pro Ala Pro
                   165                 170                 175
   Pro Ser Gly Ser Ala Pro His Phe Leu
               180                 185
<210> 2
   <211> 52
   <212> PRT
   <213> Homo sapiens
<400> 2
   Tyr Arg Gln Ser Met Asn Asn Phe Gln Gly Leu Arg Ser Phe Gly Cys
    1               5                  10                  15
   Arg Phe Gly Thr Cys Thr Val Gln Lys Leu Ala His Gln Ile Tyr Gln
               20                  25                  30
   Phe Thr Asp Lys Asp Lys Asp Asn Val Ala Pro Arg Ser Lys Ile Ser
           35                  40                  45
   Pro Gln Gly Tyr
       50
<210> 3
   <211> 48
   <212> PRT
   <213> Homo sapiens
<400> 3
   Glu Leu Arg Met Ser Ser Ser Tyr Pro Thr Gly Leu Ala Asp Val Lys
    1               5                  10                  15
   Ala Gly Pro Ala Gln Thr Leu Ile Arg Pro Gln Asp Met Lys Gly Ala
               20                  25                  30
   Ser Arg Ser Pro Glu Asp Ser Ser Pro Asp Ala Ala Arg Ile Arg Val
           35                  40                  45
<210> 4
   <211> 19
   <212> PRT
   <213> Artificial Sequence
<220>
   <223> Description of Artificial Sequence: Synthetic Peptide
<400> 4
   Cys Arg Pro Gln Asp Met Lys Gly Ala Ser Arg Ser Pro Glu Asp Ser
    1               5                  10                  15
   Ser Pro Asp
<210> 5
   <211> 13
   <212> PRT
   <213> Artificial Sequence
<220>
   <223> Description of Artificial Sequence: Synthetic Peptide
<400> 5
   Cys Ser Ser Pro Asp Ala Ala Arg Ile Arg Val Lys Arg
    1               5                  10